# FASCICULE DE BREVET EUROPEEN

(11) **EP 0 619 995 B1**
(45) Date de publication et mention de la délivrance du brevet: **31.03.1999**
(21) Numéro de dépôt: 94400790.5
(22) Date de dépôt: 12.04.1994
(51) Int. Cl.: A61G 11/00, A61G 13/10

(54) **Table médicale pourvue d'une source radiante de chauffage perfectionnée**
Ein mit einem Heizstrahler ausgerüsteter Tisch für medizinische Zwecke
A radiation heating source equipped medical table

(30) Priorité: 13.04.1993 FR 9304316
(43) Date de publication de la demande: 19.10.1994
(73) Titulaire: MEDIPREMA, F-37173 Chambray Les Tours Cédex (FR)
(72) Inventeur: Pardonge, Jean-Marc, F-37300 Joue-Les-Tours (FR)
(74) Mandataire: Ilgart, Jean-Christophe

(56) Documents cités:
- WO-A-88/04163
- DE-A- 2 308 214
- FR-A- 2 620 800
- GB-A- 2 175 213

## Description

L'invention concerne une table médicale pourvue d'une source radiante de chauffage perfectionnée.

Les tables selon l'invention peuvent servir à des applications diverses et en particulier les opérations chirurgicales ou l'incubation de nouveau-nés. Une source radiante de chauffage, qui sert éventuellement également à l'éclairage, surplombe la table et est orientée vers un matelas sur lequel la personne objet des soins est couchée. Il est essentiel de maintenir un chauffage constant quelles que soient les conditions ambiantes, notamment les fluctuations de la température ou la présence de chirurgiens ou d'autres intervenants qui peuvent intercepter une partie du rayonnement de la source.

On connaît (par exemple par le document GB 2 175 213 A, qui concerne une couveuse pour prématurés pouvant être fermée ou ouverture en haut) des sources de chauffage composées de plusieurs tubes orientés longitudinalement par rapport au matelas et mis côte à côte pour former un bloc rectangulaire de chauffage. Un thermomètre est souvent localisé près de la source pour renseigner un dispositif d'asservissement de la puissance de la source radiante, après qu'un essai préliminaire de chauffage a été mené sur un objet posé sur le matelas, et a fourni une fonction de correspondance entre des températures mesurées par le thermomètre et des températures mesurées sur l'objet ; l'asservissement consiste à maintenir la température mesurée par le thermomètre à une valeur constante qui correspond à l'échauffement de l'objet à la température du corps humain. L'objet est défini par des normes variables selon les pays afin de reproduire autant que possible les caractéristiques d'échauffement d'un nouveau-né. Il peut s'agir d'une plaque de métal noirci ou d'alliage d'aluminium recouvert de graisse puis d'un tissu blanc.

La mesure est trompeuse si le faisceau est intercepté par un intervenant, car l'occupant de la table n'est plus réchauffé correctement. Ce problème de refroidissement de l'occupant du matelas, auquel s'ajoute l'inconfort de l'intervenant exposé à la chaleur, est si gênant qu'on trouve aussi des tables de genre différent dans lesquelles le chauffage est assuré à partir du matelas. La chaleur est transmise par conduction par une petite surface du corps de l'occupant du matelas, qui doit ainsi être chauffée plus fortement. Les conséquences sont particulièrement néfastes dans le cas des opérations, surtout pour les nouveau-nés et les prématurés, car les déperditions de chaleur sont si fortes dès l'ouverture du corps, à l'endroit opposé au chauffage, que les opérations sont limitées à quelques dizaines de minutes et ne peuvent être prolongées au-delà qu'en chauffant le matelas si fortement que de graves brûlures sont inévitables.

On souhaite donc une table médicale qui soit exempte de ces inconvénients de variation et d'insuffisance de chauffage selon les circonstances extérieures. C'est le sujet de cette invention.

Un des perfectionnements proposés à titre subsidiaire consiste à ajouter un second thermomètre, en plus de celui qui est localisé près de la source, aux tables pourvues d'un dispositif d'asservissement de la puissance de la source, et ce second thermomètre est affecté à la mesure de l'air ambiant. Ce perfectionnement est utile pour les tables qui sont occupées pendant de longues périodes et ne nécessitent pas forcément de surveillance attentive, et elle peut donc trouver favorablement application à l'incubation de nouveau-nés. Comme la table conforme à l'invention est en principe dépourvue de couvercle supérieur qui permette de la transformer en couveuse en enclosant un volume abrité et en protégeant ainsi le nourrisson, il est essentiel que le second thermomètre soit sensible avant tout à l'ambiance extérieure, c'est-à-dire à sa température statique, mais aussi à l'effet de courants d'air. Il faut donc qu'il ne soit pas abrité mais extérieur et dans un endroit dégagé. Il peut être placé dans beaucoup d'endroits, mais il est préférable qu'il soit adjacent à la table, sans toutefois l'exposer au rayonnement de chauffage, ce qui réduirait sa sensibilité à l'influence des conditions ambiantes et pourrait même fausser ses résultats.

La combinaison des deux thermomètres soumis à des influences si différentes (puisque le thermomètre de mesure de la puissance de chauffage est généralement abrité) donne la possibilité de régler avec une très grande précision la température délivrée à la surface de la table.

Toutefois, le perfectionnement qui constitue l'objet essentiel de l'invention consiste à diviser la source radiante en deux blocs espacés longitudinalement par rapport au matelas ; ces blocs sont favorablement disposés en convergence vers le matelas, c'est-à-dire que leurs rayonnements forment un angle. L'intervenant se tient sous l'espace entre les blocs et n'est donc pas frappé par le rayonnement. Ce perfectionnement est appliqué le plus favorablement aux tables d'opérations chirurgicales. Ces deux perfectionnements résolvent donc

des problèmes techniques analogues (assurer un chauffage convenable et constant) tout en étant plus utiles dans des circonstances différentes, ce qui explique qu'ils peuvent parfaitement être cumulés.

L'invention va maintenant être décrite plus précisément à l'aide des figures suivantes annexées à titre illustratif et non limitatif :
- la figure 1 représente une vue générale d'une table conforme à l'invention,
- la figure 2 représente un des blocs de chauffage par sa face d'ouverture,
   et la figure 3 représente des courbes d'illustration des résultats de l'asservissement.

La table comprend donc un plan 1 de couchage soutenu par une colonne 2 montée sur un chariot à roulettes 3. Un matelas 4 est posé sur le plan 1, qui porte par ailleurs, par l'intermédiaire d'un joint tournant 5, un mât 6 vertical du sommet duquel part un bras 7 qui porte deux blocs de chauffage 8 successifs et séparés par un intervalle assez important. Le mât 6 peut être rejeté de côté pour laisser dégagé l'espace au-dessus du matelas 4 si on désire par exemple faire des radiographies. Mais quand le mât 6 est remis à sa position normale, les blocs 8 s'étendent au-dessus du matelas 4 et émettent des faisceaux orientés vers lui et qui convergent vers une zone de chauffage qui s'étend sur la plus grande partie de la surface du matelas 4 ; chacun des blocs 8 est responsable du chauffage d'une partie de cette zone. Cet effet de convergence est obtenu grâce à la courbure du bras 7, qui est cependant horizontal en moyenne. Un volume intermédiaire exempt de chauffage est donc préservé entre les blocs 8, et un intervenant tel qu'un chirurgien peut y glisser la tête et le tronc pour éviter d'être chauffé indûment et soigner la personne étendue sur le matelas 4.

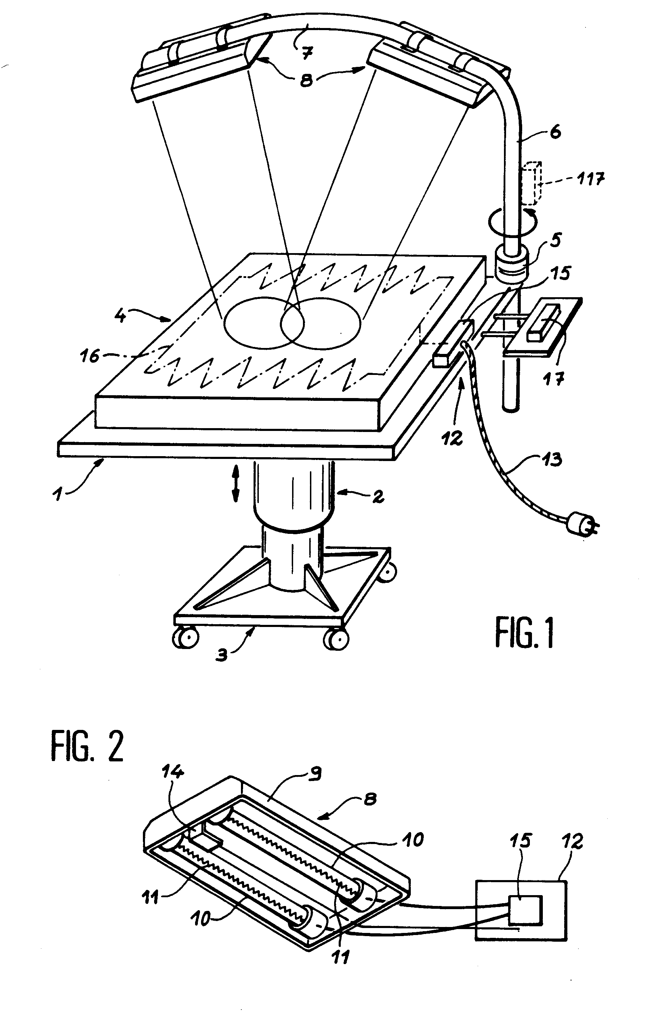

La figure 2 montre que les blocs de chauffage 8 sont composés d'un capot 9 réflecteur qui renferme des tubes 10 en quartz-silice dans lesquels un filament 11 est porté à incandescence. On pourra également utiliser d'autres moyens de chauffage tels que des lampes, des globes ou des panneaux de céramique.

Les filaments 11 sont reliés à un dispositif d'asservissement 12 qui n'est que schématisé et qui comprend en particulier un rhéostat 15 qui permet de faire varier la puissance transmise au filament 11 à partir du secteur électrique ou d'un bloc électrogène par l'intermédiaire d'un câble 13. Un thermomètre 14, fixé sous le capot 9 à proximité des tubes 10, est également prévu et relié au dispositif d'asservissement 12. Il sert à mesurer la température locale, qui dépend de l'intensité de chauffage. Il existe un thermomètre 14 pour un seul des blocs 8 ou pour les deux.

Le matelas 4 peut également contenir des résistances 16 de chauffage alimentées par le câble 13, avec ou sans la possibilité de les régler automatiquement. Elles font régner à la surface du matelas 4 une température égale à la température cutanée normale chez l'homme, ou légèrement supérieure.

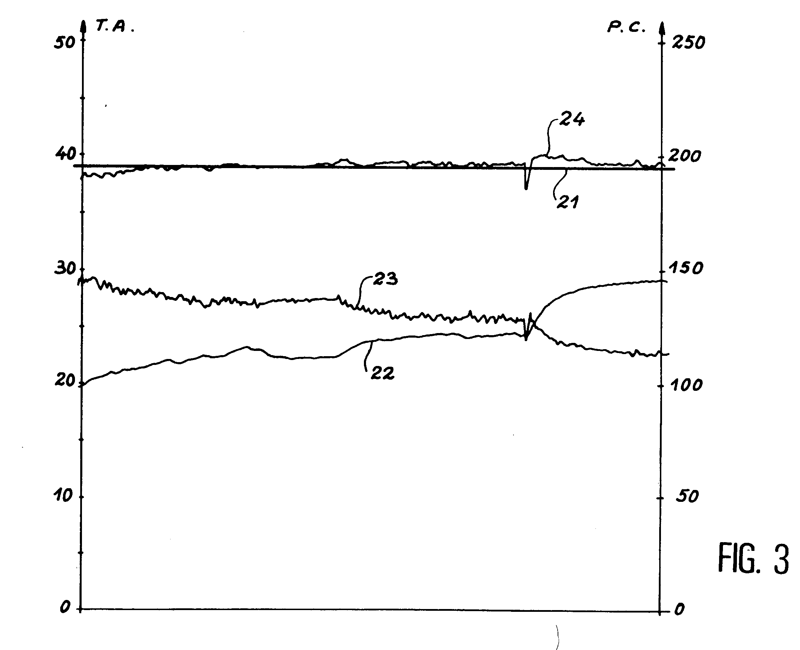

Un second thermomètre 17 est pourvu sur la table ou à côté d'elle pour mesurer la température de l'air ambiant, afin de corriger son influence sur la température de chauffage, qui est assez sensible comme on le voit sur les courbes de la figure 3, qui représente des évolutions de température en fonction du temps. Si un asservissement à 39°C (droite 21) est décidé sur le matelas 4 et que l'air ambiant s'échauffe de 20°C à 29°C environ (courbe 22), le chauffage doit être modéré en conséquence : la température mesurée par le thermomètre 14 s'abaisse de 145°C à 120°C environ (courbe 23) pour donner finalement la courbe 24, très peu différente de la droite 21 et qui exprime la température mesurée sur un objet normalisé de simulation dont on a déjà parlé. On constate à la fois l'importance de l'influence de la température de l'air ambiant et la bonne qualité de l'asservissement réalisé.

L'influence de la température de l'air ambiant intervient dans le réglage du rhéostat 15 sous forme d'une action correctrice définie par une tabulation préliminaire ingérée par l'ordinateur du dispositif d'asservissement 12 : la température demandée à l'endroit du thermomètre 14 est déterminée en fonction de la température de l'air ambiant et de la température de chauffage souhaitée sur le matelas 4. L'emplacement du second thermomètre 17 n'est pas critique à condition qu'il ne soit pas atteint par le rayonnement des blocs 8 de chauffage : on l'a figuré à une extrémité de la table, sur une tablette adjacente, mais bien d'autres endroits sont possibles, et il est même concevable de le séparer physiquement de la table pourvu qu'il reste relié électriquement au dispositif d'asservissement 12. Il est envisagé également de l'accrocher au mât 6, à une hauteur relativement faible pour qu'il soit à peu près à hauteur du nourrisson et éloigné des blocs 8 de chauffage. Dans une telle réalisation, il serait placé selon la référence 117.

Ces moyens de régulation rendent l'invention attrayante pour toutes les applications où la stabilisation du chauffage à la température du corps humain est indispensable et surtout pour les nourrissons et les enfants prématurés, qui peuvent être couvés sans risque de perturbation et opérés pendant des durées assez longues s'il le faut. L'invention peut cependant être appliquée aussi aux tables pour adultes.

## Revendications

1. Table médicale comprenant un matelas (4) et une source radiante de chauffage orientée vers le matelas, caractérisée en ce que la source est divisée en deux blocs (8) espacés en direction longitudinale de la table.

2. Table médicale suivant la revendication 1, caractérisé en ce que les blocs sont disposés de façon que leurs rayonnements convergent.

3. Table médicale suivant l'une quelconque des revendications 1 ou 2, caractérisée en ce que les sources sont essentiellement composées de tubes (11) en quartz-silice enfermant un filament.

4. Table médicale suivant l'une quelconque des revendications 1 à 3, caractérisée en ce que le matelas est muni de moyens additionnels de chauffage (16).

5. Table médicale suivant l'une quelconque des revendications 1 à 4, caractérisée en ce qu'elle est appliquée aux opérations chirurgicales.

6. Table médicale suivant l'une quelconque des revendications précédentes 1 à 4 et pourvue en outre d'un dispositif d'asservissement de la puissance (12) de la source radiante ainsi que d'un thermomètre (14) localisé près de la source, caractérisée en ce qu'elle comprend un second thermomètre (17) affecté à la mesure de la température de l'air ambiant et placé hors du rayonnement de la source et à côté de la table, les thermomètres étant reliés au dispositif d'asservissement pour aider à ajuster la puissance de la source radiante.

7. Table médicale suivant la revendication 6, caractérisée en ce qu'elle est appliquée à l'incubation de nouveau-nés.

## Claims

1. Medical table comprising a mattress (4) and a radiating source of heat oriented towards the mattress, characterized in that the source is divided into two blocks (8) which are spaced apart in the longitudinal direction of the table.

2. Medical table according to Claim 1, characterized in that the blocks are arranged in such a way that their rays converge.

3. Medical table according to either of Claims 1 and 2, characterized in that the sources are essentially composed of tubes (11) of quartz-silica enclosing a filament.

4. Medical table according to any one of Claims 1 to 3, characterized in that the mattress is equipped with additional heating means (16).

5. Medical table according to any one of Claims 1 to 4, characterized in that it is used in surgical operations.

6. Medical table according to any one of the preceding Claims 1 to 4 and provided in addition with a power control device (12) for controlling the radiating source, and with a thermometer (14) located near the source, characterized in that it comprises a second thermometer (17) assigned to measure the temperature of the ambient air and placed outside the rays of the source and alongside the table, the thermometers being connected to the control device in order to aid in adjusting the power of the radiating source.

7. Medical table according to Claim 6, characterized in that it is used for incubation of neonates.

## Patentansprüche

1. Medizinischer Tisch mit einer Matraze (4) und einem auf diese Matratze gerichteten Heizstrahler,
**dadurch gekennzeichnet**,
daß der Heizstrahler verteilt ist auf zwei in Längsrichtung des Tisches voneinander beabstandete Blöcke (8).

2. Medizinischer Tisch nach Anpruch 1, dadurch gekennzeichnet, daß die Blöcke so angeordnet sind, daß ihre Stahlungen konvergieren.

3. Medizinischer Tisch nach einem der Anprüche 1 oder 2, dadurch gekennzeichnet, daß die Heizstrahler im wesentlichen durch Quarz-Siliciumdioxid-Röhren bzw. Quarzglas-Röhren (11) gebildet werden, die einen Glühdraht bzw. -faden enthalten.

4. Medizinischer Tisch nach einem der Anprüche 1 bis 3, dadurch gekennzeichnet, daß die Matratze mit zusätzlichen Heizeinrichtungen (16) ausgerüstet ist.

5. Medizinischer Tisch nach einem der Anprüche 1 bis 4, dadurch gekennzeichnet, daß er für chirurgische Operationen benutzt wird.

6. Medizinischer Tisch nach einem der vorangehenden Anprüche 1 bis 4 und zudem mit einer Regeleinrichtung (12) der Leistung des Heizstrahlers sowie einem nahe bei diesem Heizstrahler befindlichen Thermometer (14) ausgestattet, dadurch gekennzeichnet, daß er ein zweites Thermometer (17) zum Messen der Umgebungsluft umfaßt, das außerhalb der Strahlung des Heizstrahlers und seitlich des Tisches angeordnet ist, wobei die Thermometer mit der Regeleinrichtung verbunden sind, um die Leistung des Heizstrahlers zu regulieren.

7. Medizinischer Tisch nach Anpruch 6, dadurch gekennzeichnet, daß er als Bruteinrichtung für Neugeborene dient.
